# EUROPÄISCHE PATENTSCHRIFT

(11) **EP 3 072 544 B1**
(45) Veröffentlichungstag und Bekanntmachung des Hinweises auf die Patenterteilung: **27.02.2019**
(21) Anmeldenummer: 15160380.0
(22) Anmeldetag: 23.03.2015
(51) Int. Cl.: A61M 1/10, A61M 1/12

(54) **SYSTEM ZUR VENTRIKULÄREN KREISLAUFUNTERSTÜTZUNG**
SYSTEM PROVIDING VENTRICULAR CIRCULATORY SUPPORT
SYSTÈME D'ASSISTANCE CIRCULATOIRE VENTRICULAIRE

(43) Veröffentlichungstag der Anmeldung: 28.09.2016
(73) Patentinhaber: Ensminger, Stephan, 91080 Marloffstein (DE)
(72) Erfinder: Ensminger, Stephan, 91080 Marloffstein (DE); Kaufmann, Tim, 52070 Aachen (DE)
(74) Vertreter: Trinks, Ole

(56) Entgegenhaltungen:
- EP-A1- 0 760 244
- WO-A1-02/26292
- GB-A- 2 323 215
- US-A1- 2010 280 303

## Beschreibung

Die Erfindung betrifft ein System zur ventrikulären Kreislaufunterstützung.
Bekannte Systeme zur ventrikulären Kreislaufunterstützung, insbesondere Kunstherzen, sind als mechanische Pumpen ausgebildet (siehe e.g. EP0760244). Hierbei kommen diverse Systeme zum Einsatz, beispielsweise Kreiselpumpen, kontinuierlich arbeitende Radial- oder Axialpumpen, pulsartige Verdrängungspumpen etc., wobei zum Antrieb starre mechanische Verbindungen oder pneumatische Verbindungen bekannt sind. Diese bekannten Systeme kommen entweder zur Unterstützung des Herzens zum Einsatz, in der Regel als Linksherzunterstützungssystem (LVAD) oder Rechtsherzunterstützungssystem (RVAD).
In Extremfällen müssen beide Herzkammern unterstützt werden (biventrikuläres Unterstützungssystem (BiVAD)). Beim Ausfall des natürlichen Herzens kann es auch notwendig sein, ein Kunstherz als vollständigen Ersatz des natürlichen Herzens vorzusehen (total artificial heart TAH). Hierbei wird in einer Operation das natürliche Herz des Patienten vollständig explantiert und durch eine entsprechende mechanische Pumpe ersetzt. Alternativ kann das Kunstherz extrakorporal vorgesehen sein.
Bekannte Systeme weisen die Nachteile auf, dass die mechanischen Bestandteile des Kunstherzens sowohl bei einem unterstützenden Kunstherz als auch bei einem Kunstherz, welches zum vollständigen Ersatz des natürlichen Herzens vorgesehen ist, Verschleiß und Reibung ausgesetzt sind. Hierdurch wird die Lebensdauer eines solchen Systems limitiert und ein derartiges Kunstherz muss nach relativ kurzer Zeit ersetzt werden. Auch wird die Funktionssicherheit eines derartigen Kunstherzens gefährdet, was im Falle des Versagens des Kunstherz für einen Patienten lebensbedrohlich sein kann.

Zusätzlich ist für alle derzeit erhältliche Kunstherzen als Folge des Blutkontaktes mit der künstlichen Oberfläche des Systems eine strenge Antikoagulation (Blutverdünnung) der Patienten notwendig, damit es nicht zu Blutgerinnseln kommen kann. Diese Notwendigkeit ist für einen substantiellen Anteil an Komplikationen wie z.B. Apoplex oder Gehirnblutung während einer Therapie mit einem Kunstherzsystem verantwortlich.

Die Aufgabe der Erfindung ist es daher, ein System zur ventrikulären Kreislaufunterstützung anzugeben, welches zuverlässiger und langlebiger ist.

Die erfindungsgemäße Aufgabe wird durch den Gegenstand des Patentanspruchs 1 gelöst. Vorteilhafte Weiterbildungen sind in den abhängigen Patentansprüchen angegeben.

Demgemäß wird ein System zur ventrikulären Kreislaufunterstützung vorgeschlagen, welches eine Einrichtung zum Applizieren eines Magnetfeldes auf eine magnetisierte Körperflüssigkeit, insbesondere Blut in einen Bereich eines Gefäßes, insbesondere Blutgefäßes aufweist. Das System weist darüber hinaus eine Steuereinrichtung auf, welche ausgebildet ist, die Einrichtung zum Applizieren eines Magnetfeldes derart anzusteuern, dass in dem Bereich des Gefäßes auf die magnetisierte Körperflüssigkeit eine magnetisch induzierte Kraft in Längsrichtung des Gefäßes einwirkt.

Die erfindungsgemäßen Vorteile liegen auf der Hand. Während bei mechanischen Systemen durch die ständig benötigte Pumpleistung des ventrikulären Kreislaufunterstützungssystems Verschleiß unvermeidlich ist und die Funktionssicherheit gefährdet ist, ist bei dem erfindungsgemäßen System zur ventrikulären Kreislaufunterstützung der Verschleiß im Vergleich zu bekannten Systemen reduziert, da keine beweglichen mechanischen Elemente notwendig sind. Aus demselben Grund kann die Betriebssicherheit erhöht werden, da keine mechanischen und insbesondere mechanisch beweglichen Teile des Systems zur ventrikulären Kreislaufunterstützung bewegt werden müssen.

Insbesondere zeichnet sich die erfindungsgemäße Lösung dadurch aus, dass das System zur ventrikulären Kreislaufunterstützung außerhalb des Blutgefäßes angeordnet sein kann, und somit ganz ohne direkten Blutkontakt arbeitet. Von daher kann auf eine Blutverdünnung verzichtet werden.

Gemäß einem Aspekt der Erfindung ist vorgesehen, dass die Steuereinrichtung ferner ausgebildet ist, die Stärke und/oder die Richtung des auf die magnetisierte Körperflüssigkeit applizierten Magnetfeldes derart zeitlich zu variieren, dass sich die Körperflüssigkeit wahlweise kontinuierlich oder pulsartig in Längsrichtung des Gefäßes bewegt. Zusätzlich ist auch eine helikale Bewegung von Körperflüssigkeiten möglich.

Durch ein kontinuierliches Strömungsverhalten der Körperflüssigkeit kann eine gleichmäßige Versorgung des Körpers eines Patienten mit der Körperflüssigkeit gewährleistet werden. Insbesondere in dem Fall, in dem es sich bei der Körperflüssigkeit um Blut handelt, kann gewährleistet werden, dass alle Körperteile des Patienten gleichmäßig mit Blut versorgt werden. Vorteilhafterweise führt die kontinuierliche Strömung des Blutes in diesem Fall auch dazu, dass Gefäße entlastet werden, insbesondere dadurch, dass keine wiederkehrenden Druckschwankungen auf die Gefäßwände einwirken.

In diesem Zusammenhang sei angemerkt, dass nicht nur Blut durch das ventrikuläre Kreislaufunterstützungssystem bewegt werden kann. Beispielsweise kann es sich bei der Körperflüssigkeit auch um Lymphflüssigkeit handeln.

Alternativ kann die Steuereinrichtung das auf die magnetisierte Körperflüssigkeit applizierte Magnetfeld auch so zeitlich variieren, dass die Körperflüssigkeit pulsartig in Längsrichtung der Gefäßes bewegt wird. In diesem Fall wird die natürliche Funktionalität eines natürlichen Herzen simuliert. Somit kann erreicht werden, dass alle Funktionen des Körpers eines Patienten in gewohnter Weise weiterbetrieben werden können, da das System zur ventrikulären Kreislaufunterstützung das natürliche Herz eines Patienten funktional ersetzt.

Gemäß einem weiteren Aspekt der Erfindung ist vorgesehen, dass das System zur ventrikulären Kreislaufunterstützung ferner eine Magnetisiereinrichtung zum Magnetisieren einer Körperflüssigkeit, insbesondere Blut, aufweist.

Hierdurch wird gewährleistet, dass die Körperflüssigkeit optimal durch das Applizieren des Magnetfeldes bewegt werden kann, d. h. dass auf optimierte Weise eine Kraft auf die Körperflüssigkeit übertragen werden kann.

In diesem Zusammenhang sei angemerkt, dass die Körperflüssigkeiten eines Patienten, insbesondere Blut, korpuskulare Bestandteile wie beispielsweise Erythrozyten aufweist. Durch das Applizieren eines Magnetfeldes kann insbesondere dann eine magnetische Kraft ausgeübt werden, wenn die korpuskularen Bestandteile magnetisiert wurden.

Gemäß einem weiteren Aspekt der Erfindung ist vorgesehen, dass die Magnetisiereinrichtung ausgebildet ist, magnetisierte oder magnetisierbare Partikel in die Körperflüssigkeit zu impfen.

Hierdurch kann die Magnetisierung der Körperflüssigkeit, insbesondere durch die Magnetisierung von in der Körperflüssigkeit enthaltenen Bestandteilen, erhöht werden.

Denkbar ist hierbei, dass beispielsweise magnetisierte oder magnetisierbare Partikel, beispielsweise eisenhaltige Partikel, zumindest temporär an in der Körperflüssigkeit enthaltene Zellen angelagert werden. Diese Anlagerung kann beispielsweise durch einen chemischen Prozess durchgeführt werden. Denkbar ist auch, dass eine derartige Anlagerung dadurch erreicht wird, dass die Oberfläche der magnetischen oder magnetisierbaren Partikel so modifiziert wird, dass diese Partikel an die in der Körperflüssigkeit enthaltenen Zellen "andocken". Denkbar ist hierbei eine Bindung analog dem Schlüssel-Schloss-Prinzip ähnlich der Bindung von Antikörpern an entsprechende Viren.

Andererseits ist denkbar, dass die magnetischen oder magnetisierbaren Partikel als Nanopartikel ausgebildet sind und beispielsweise innerhalb von in der Körperflüssigkeit befindlichen Zellen eingeschlossen werden. Auch ist natürlich denkbar, dass die magnetischen oder magnetisierbaren Partikel frei im Blutplasma bzw. in der Körperflüssigkeit vorgesehen werden.

Gemäß einem weiteren Aspekt der Erfindung ist vorgesehen, dass die Magnetisiereinrichtung ausgebildet ist, die Körperflüssigkeit derart zu behandeln, dass bei Bestandteilen der Körperflüssigkeit ein Dipolmoment ausgebildet wird.

Durch das Ausbilden eines Dipolmoments wird ermöglicht, durch Applizieren eines Magnetfeldes eine magnetische Kraft auf eben diese Bestandteile der Körperflüssigkeit auszuüben.

Gemäß einem weiteren Aspekt der Erfindung ist vorgesehen, dass die Magnetisiereinrichtung ausgebildet ist, intrakorporal die Körperflüssigkeit zu magnetisieren, insbesondere durch orale Verabreichung oder durch intravenöse Injektion von magnetisierten oder magnetisierbaren Partikeln.

Denkbar ist in diesem Fall, dass magnetisierte oder magnetisierbare Partikel in Tablettenform oral verabreicht werden oder durch eine Spritze injiziert werden. Vorzugsweise werden die Partikel durch intravenöse Injektion direkt dem Blutkreislauf eines Patienten zugeführt, sodass innerhalb kurzer Zeit das Blut des Patienten magnetisiert oder magnetisierbar ist.

Gemäß einem weiteren Aspekt der Erfindung ist vorgesehen, dass die Magnetisiereinrichtung ausgebildet ist, extrakorporal die Körperflüssigkeit zu magnetisieren, insbesondere durch Injektion oder Diffusion von magnetisierten oder magnetisierbaren Partikeln.

Denkbar ist hierbei, dass die Magnetisierung der Körperflüssigkeit beispielsweise bei einer Dialyse des Blutes eines Patienten durchgeführt wird. Zu diesem Zweck kann ein Dialysegerät verwendet werden oder aber ein Dialysegerät modifiziert werden, sodass dieses Dialysegerät zur Magnetisierung der Körperflüssigkeit eines Patienten ausgelegt ist. Andererseits ist natürlich denkbar, dass zu diesem Zweck kein Dialysegerät zum Einsatz kommt, sondern ein anderes Gerät, welches ausgelegt ist, magnetisierbare oder magnetisierte Partikel in das Blut eines Patienten zu injizieren oder durch Diffusion von magnetisierten oder magnetisierbaren Partikeln in die Körperflüssigkeit eines Patienten die Körperflüssigkeit zu magnetisieren.

Gemäß einem weiteren Aspekt der Erfindung ist vorgesehen, dass die Einrichtung zum Applizieren eines Magnetfeldes zumindest bereichsweise in Längsrichtung des Gefäßes eine Magnetspulenanordnung aufweist, welche in mehrere aufeinander folgende und unabhängig voneinander ansteuerbare Bereiche segmentiert ist.

Die Magnetspulenanordnung besteht vorzugsweise aus zumindest einem Elektromagneten, welcher ausgelegt ist, bei Aktivierung ein Magnetfeld zu erzeugen, welches geeignet ist, mindestens einen ein Dipolmoment aufweisenden Partikel innerhalb der Körperflüssigkeit eines Patienten in Längsrichtung des Gefäßes zu beschleunigen.

Gemäß einem weiteren Aspekt der Erfindung ist vorgesehen, dass die Steuereinrichtung ausgebildet ist, in Flussrichtung der Körperflüssigkeit nacheinander die einzelnen Bereiche der Magnetspulenanordnung derart anzusteuern, dass jeweils von einem einzelnen Bereich allein ein Magnetfeld auf die magnetisierte Flüssigkeit appliziert wird.

Auf diese Weise wird erreicht, dass Partikel, welche in der Körperflüssigkeit des Patienten enthalten sind und welche ein Dipolmoment besitzen, in Flussrichtung beschleunigt werden können und somit erreicht wird, dass die Körperflüssigkeit des Patienten in Flussrichtung des Gefäßes bewegt wird. Hierbei wird der Effekt ausgenutzt, dass durch interne Reibung die Körperflüssigkeit als Ganzes in Bewegung versetzt wird, selbst wenn nur einzelne Bestandteile, welche in der Körperflüssigkeit enthalten sind, beschleunigt werden. In anderen Worten "reißen" die Bestandteile, welche ein Dipolmoment besitzen und durch die Magnetspulenanordnung beschleunigt werden, die restlichen, nicht magnetisierten, Bestandteile der Körperflüssigkeit mit, sodass insgesamt eine Bewegung der Körperflüssigkeit eines Patienten in Flussrichtung des Gefäßes realisiert wird.

Gemäß einem weiteren Aspekt der Erfindung ist vorgesehen, dass die einzelnen Bereiche der Magnetspulenanordnung räumlich voneinander getrennt sind, und wobei in Strömungsrichtung der Körperflüssigkeit gesehen der erste Bereich der Magnetspulenanordnung von der Steuereinrichtung ansteuerbar ist zum Ausüben eines magnetischen Moments auf stromaufwärts des ersten Bereiches angeordnete magnetisierte oder magnetisierbare Partikel, und wobei ein im Hinblick auf den ersten Bereich der Magnetspulenanordnung stromabwärts angeordneter weiterer Bereich der Magnetspulenanordnung von der Steuereinrichtung angesteuert wird, wenn die magnetisierten oder magnetisierbaren Partikel durch das Magnetfeld des ersten Bereiches der Magnetspulenanordnung entlang des Gefäßes in Richtung des ersten Bereiches der Magnetspulenanordnung bewegt wurde.

Auf diese Weise können die Bestandteile der Körperflüssigkeit, die ein Dipolmoment aufweisen, mehrere mal einer Beschleunigung und vorzugsweise der gleichen Beschleunigung ausgesetzt werden. Denkbar ist, dass hierdurch eine lineare Beschleunigung auf die magnetischen oder magnetisierbaren Bestandteile der Körperflüssigkeit ausgeübt wird. Die Bereiche der Magnetspulenanordnung können in anderen Worten so angesteuert werden, dass die Körperflüssigkeit linear und gleichmäßig beschleunigt wird, sodass eine kontinuierliche Kraft auf die magnetisierten oder magnetisierbaren Bestandteile der Körperflüssigkeit ausgeübt wird. Somit wird eine konstante Pumpleistung erzeugt.

Andererseits können die Bereiche der Magnetspulenanordnung so angesteuert werden, dass eine pulsartige oder wellenartige Kraft auf die magnetischen oder magnetisierbaren Bestandteile der Körperflüssigkeit ausgeübt werden. Hierdurch kann die natürliche Funktionalität eines Herzens simuliert werden.

Gemäß einem weiteren Aspekt der Erfindung ist vorgesehen, dass die Einrichtung zum Applizieren eines Magnetfeldes eine Vielzahl von Magnetspulenanordnungen in aufeinanderfolgenden Gefäßabschnitten aufweist, und wobei entlang der aufeinanderfolgenden Gefäßabschnitte ein von der Einrichtung appliziertes Magnetfeld derart generiert wird, dass auf magnetisierte oder magnetisierbare Partikel in der Körperflüssigkeit eine Magnetkraft ausgeübt wird, die eine in Längsrichtung des Gefäßes zeigende Komponente aufweist.

Hierdurch kann eine einzelne Magnetspulenanordnung kleiner dimensioniert werden und gleichzeitig eine vergleichbare ventrikuläre Kreislaufunterstützung erzielt werden.

Nachfolgend wird die Erfindung unter Bezugnahme auf die beiliegenden Zeichnungen anhand exemplarischer Ausführungsformen des erfindungsgemäßen Systems zur ventrikulären Kreislaufunterstützung näher beschrieben.

In den Zeichnungen zeigen:
- FIG. 1:: eine schematische Schnittansicht des erfindungsgemäßen Systems zur ventrikulären Kreislaufunterstützung, eines Gefäßes sowie eines magnetischen oder magnetisierbaren Partikels;
- FIG. 2:: eine schematische Schnittansicht des erfindungsgemäßen Systems zur ventrikulären Kreislaufunterstützung, eines Gefäßes sowie eines magnetischen oder magnetisierbaren Partikels mit Ausübung einer magnetischen Kraft durch einen zweiten Bereich einer Magnetspulenanordnung;
- FIG. 3:: eine schematische Schnittansicht des erfindungsgemäßen Systems zur ventrikulären Kreislaufunterstützung, eines Gefäßes sowie eines magnetischen oder magnetisierbaren Partikels mit Ausübung einer magnetischen Kraft durch einen dritten Bereich einer Magnetspulenanordnung; und
- FIG. 4:: eine schematische Schnittansicht des erfindungsgemäßen Systems zur ventrikulären Kreislaufunterstützung, eines Gefäßes sowie zwei magnetische oder magnetisierbare Partikel.

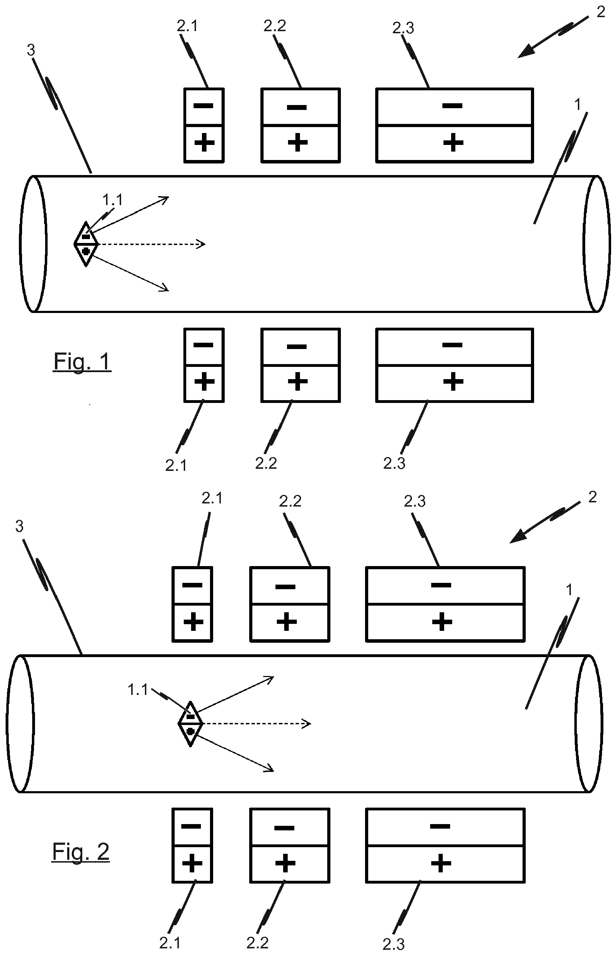

FIG. 1 zeigt eine Einrichtung 2 zum Applizieren eines Magnetfeldes auf eine magnetisierte Körperflüssigkeit 1. Die Einrichtung 2 ist als Magnetspulenanordnung mit exemplarischen drei Bereichen 2.1, 2.2, 2.3 ausgebildet, die ein Gefäß 3 umgibt.

Innerhalb des Gefäßes 3 befindet sich ein Bestandteil 1.1, wie ein magnetischer oder magnetisierbarer Partikel oder eine magnetisierte oder magnetisierbare Zelle, einer Körperflüssigkeit 1 eines Patienten. In FIG. 1 ist schematisch lediglich ein einziger derartiger Bestandteil 1.1 gezeigt. Natürlich sind im Regelfall innerhalb der Körperflüssigkeit 1 eines Patienten viele Bestandteile, die magnetisiert oder magnetisierbar sind, enthalten.

Die Magnetspulenanordnung 2 ist in FIG. 1 schematisch aus drei Bereichen 2.1, 2.2, 2.3 zusammengesetzt. Natürlich ist denkbar, dass die Magnetspulenanordnung 2 lediglich über einen Bereich oder aber über mehr als drei Bereiche verfügt. In FIG. 1 schematisch dargestellt ist der Fall, dass ein Bestandteil 1.1, der magnetisiert oder magnetisierbar ist und in der Körperflüssigkeit 1 eines Patienten vorgesehen ist, in die Nähe der Magnetspulenanordnung gelangt.

Es wird ein erster Bereich 2.1 der Magnetspulenanordnung 2 durch eine Steuereinrichtung (nicht abgebildet) angesteuert derart, dass der Bestandteil 1.1 eine Kraft in Längsrichtung und in Flussrichtung des Gefäßes 3 versprüht. Wie in FIG. 1 dargestellt, wirkt auf den Bestandteil 1.1, der magnetisiert oder magnetisierbar ist, in diesem Fall eine magnetische Kraft vom Minuspol des Dipols des Bestandteils 1.1 auf den Pluspol des ersten Bereichs 2.1 der Magnetspulenanordnung 2 und eine Kraft von dem Pluspol des Bestandteils 1.1 auf den Minuspol des ersten Bereichs 2.1 der Magnetspulenanordnung 2.

In diesem Zusammenhang sei angemerkt, dass der Begriff "Minuspol" und "Pluspol" als magnetischer Nord- bzw. Südpol verstanden werden soll.

Durch die auf den Bestandteil 1.1 wirkenden magnetischen Kräfte (Pfeile mit durchgezogenen Linien, ausgehend vom Bestandteil 1.1 in FIG. 1) wird eine resultierende Kraft (Pfeil mit gestrichelter Linie ausgehend vom Bestandteil 1.1 in FIG. 1) auf den Bestandteil 1.1 ausgeübt, die eine Beschleunigung des Bestandteils 1.1 in Flussrichtung innerhalb des Gefäßes 3 erzeugt.

In FIG. 2 gezeigt ist der Fall, dass der Bestandteil 1.1 innerhalb des Gefäßes 3 in Flussrichtung beschleunigt wurde und nunmehr die Magnetspulenanordnung 2 derart angesteuert wird, dass der zweite Bereich 2.2 der Magnetspulenanordnung 2 angesteuert wird, sodass, wie bereits in FIG. 1 beschrieben, nunmehr entsprechend vom zweiten Bereich 2.2 der Magnetspulenanordnung 2 eine Kraft auf den magnetischen oder magnetisierbaren Bestandteil 1.1 in der Körperflüssigkeit 1 in Längsrichtung bzw. in Flussrichtung des Gefäßes 3 ausgeübt wird.

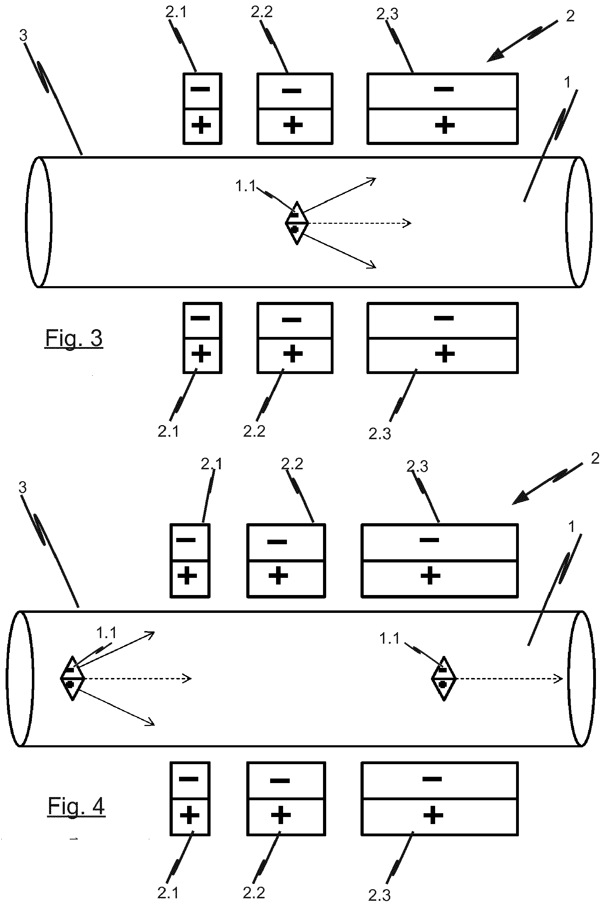

FIG. 3 zeigen den entsprechenden Fall, indem der magnetische oder magnetisierbare Bestandteil 1.1 innerhalb der Körperflüssigkeit 1 des Patienten weiter in Flussrichtung des Gefäßes 3 beschleunigt wurde und nunmehr der dritte Bereich 2.3 der Magnetspulenanordnung 2 von der Steuereinrichtung angesteuert wird, sodass dieser dritte Bereich 2.3 der Magnetspulenanordnung 2 eine magnetische Kraft auf den magnetischen oder magnetisierbaren Bestandteil 1.1 in Flussrichtung des Gefäßes 3 ausübt.

FIG. 4 zeigt wiederum den Fall, dass ein weiteres magnetisches oder magnetisierbares Partikel 1.1 bzw. in weiterer magnetischer oder magnetisierbarer Bestandteil 1.1 der Körperflüssigkeit 1 des Patienten sich der Magnetspulenanordnung 2 nähert und eine entsprechende Steuereinrichtung die Bereiche 2.1, 2.2, 2.3 der Magnetspulenanordnung 2 ansteuert, sodass auch dieser Bestandteil 1.1 in Flussrichtung des Gefäßes 3 beschleunigt wird.

Funktional wird also in anderen Worten die Magnetspulenanordnung 2 angesteuert derart, dass die Bestandteile 1.1 der Körperflüssigkeit 1 des Patienten vorzugsweise linear in Flussrichtung des Gefäßes 3 beschleunigt werden.

Andererseits können die Bereiche 2.1, 2.2, 2.3 der Magnetspulenanordnung 2 so angesteuert werden, dass eine pulsartige Beschleunigung der Bestandteile 1.1 innerhalb der Körperflüssigkeit 1 des Patienten in Flussrichtung des Gefäßes 3 erzeugt wird.

Die Erfindung ist nicht auf die in den Zeichnungen dargestellten Ausführungsformen des Systems zur ventrikulären Kreislaufunterstützung beschränkt, sondern ergibt sich aus einer Zusammenschau sämtlicher hierin offenbarter Merkmale.

### Bezugszeichenliste

- 1: magnetisierte Körperflüssigkeit
- 1.1: Bestandteil/Partikel
- 2: Einrichtung P/Magnetspulenanordnung
- 2.1, 2.2, 2,3: Bereiche der Magnetspulenanordnung
- 3: Gefäß

## Patentansprüche

1. System zur ventrikulären Kreislaufunterstützung, wobei das System folgendes aufweist:
- eine Einrichtung (2) zum Applizieren eines Magnetfeldes auf eine magnetisierte Körperflüssigkeit (1), insbesondere Blut, in einem Bereich eines Gefäßes (3), insbesondere Blutgefäßes; und
- eine Steuereinrichtung, welche ausgebildet ist, die Einrichtung (2) zum Applizieren eines Magnetfeldes derart anzusteuern, dass in dem Bereich des Gefäßes (3) auf die magnetisierte Körperflüssigkeit (1) eine magnetisch induzierte Kraft in Längsrichtung des Gefäßes einwirkt.

2. System nach Anspruch 1, wobei die Steuereinrichtung ferner ausgebildet ist, die Stärke und/oder Richtung des auf die magnetisierte Körperflüssigkeit (1) applizierten Magnetfeldes derart zeitlich zu variieren, dass sich die Körperflüssigkeit (1) vorzugsweise wahlweise kontinuierlich oder pulsartig in Längsrichtung des Gefäßes (3) bewegt.

3. System nach Anspruch 1 oder 2, welches ferner eine Magnetisiereinrichtung zum Magnetisieren einer Körperflüssigkeit (1), insbesondere Blut, aufweist.

4. System nach Anspruch 3, wobei die Magnetisiereinrichtung ausgebildet ist, magnetisierte oder magnetisierbare Partikel (1.1) in die Körperflüssigkeit (1) zu impfen.

5. System nach Anspruch 3, wobei die Magnetisiereinrichtung ausgebildet ist, die Körperflüssigkeit (1) derart zu behandeln, dass bei Bestandteilen (1.1) der Körperflüssigkeit (1) ein Dipolmoment ausgebildet wird.

6. System nach einem der Ansprüche 3 bis 5, wobei die Magnetsiereinrichtung ausgebildet ist, extravasal, intravasal oder intrakorporal die Körperflüssigkeit (1) zu magnetisieren, insbesondere durch orale Verabreichung oder durch intravenöse Injektion von magnetisierten oder magnetisierbaren Partikeln (1.1).

7. System nach einem der Ansprüche 3 bis 5, wobei die Magnetisiereinrichtung ausgebildet ist, extrakorporal die Körperflüssigkeit (1) zu magnetisieren, insbesondere durch Injektion oder Diffusion von magnetisierten oder magnetisierbaren Partikeln (1.1).

8. System nach einem der Ansprüche 1 bis 7, wobei die Einrichtung (2) zum Applizieren eines Magnetfeldes eine zumindest bereichsweise in Längsrichtung des Gefäßes (3) angeordnete Magnetspulenordnung aufweist, welche in mehrere aufeinanderfolgende und unabhängig voneinander ansteuerbare Bereiche (2.1, 2.2, 2.3) segmentiert ist.

9. System nach Anspruch 8, wobei die Steuereinrichtung ausgebildet ist, in Flussrichtung der Körperflüssigkeit (1) nacheinander die einzelnen Bereiche (2.1, 2.2, 2.3) der Magnetspulenanordnung derart anzusteuern, dass jeweils von einem einzelnen Bereich (2.1, 2.2, 2.3) allein ein Magnetfeld auf die magnetisierte Körperflüssigkeit (1) appliziert wird.

10. System nach Anspruch 8 oder 9,
wobei die einzelnen Bereiche (2.1, 2.2, 2.3) der Magnetspulenanordnung räumlich voneinander getrennt sind, und wobei in Strömungsrichtung der Körperflüssigkeit (1) gesehen der erste Bereich (2.1) der Magnetspulenanordnung von der Steuereinrichtung ansteuerbar ist zum Ausüben eines magnetischen Moments auf stromaufwärts des ersten Bereiches angeordnete magnetisierte oder magnetisierbare Partikel (1.1), und wobei ein im Hinblick auf den ersten Bereich (2.1) der Magnetspulenanordnung stromabwärts angeordneter weiterer Bereich (2.2, 2.3) der Magnetspulenanordnung von der Steuereinrichtung angesteuert wird, wenn die magnetisierten oder magnetisierbaren Partikel (1.1) durch das Magnetfeld des ersten Bereiches (2.1) der Magnetspulenanordnung entlang des Gefäßes (3) in Richtung des ersten Bereiches (2.1) der Magnetspulenanordnung bewegt wurde.

11. System nach einem der Ansprüche 1 bis 10, wobei die Einrichtung (2) zum Applizieren eines Magnetfeldes eine Vielzahl von Magnetspulenanordnungen in aufeinanderfolgenden Gefäßabschnitten aufweist, und wobei entlang der aufeinanderfolgenden Gefäßabschnitte ein von der Einrichtung (2) appliziertes Magnetfeld derart generiert wird, dass auf magnetisierte oder magnetisierbare Partikel (1.1) in der Körperflüssigkeit (1) eine Magnetkraft ausgeübt wird, die eine in Längsrichtung des Gefäßes (3) zeigende Komponente aufweist.

12. System nach einem der Ansprüche 1 bis 11, wobei zumindest die Einrichtung (2) zum Applizieren eines Magnetfeldes in den Körper eines Patienten implantierbar ist.

## Claims

1. A system for ventricular circulatory support, wherein the system comprises the following:
- a device (2) for applying a magnetic field to a magnetized bodily fluid (1), particularly blood, in a region of a vessel (3), particularly a blood vessel; and
- a control device designed to actuate the device (2) for applying a magnetic field such that a magnetically induced force acts on the magnetized bodily fluid (1) in the longitudinal direction of the vessel in the region of said vessel (3).

2. The system according to claim 1, wherein the control device is further designed to vary the strength and/or direction of the magnetic field applied to the magnetized bodily fluid (1) over time such that the bodily fluid (1) moves in the longitudinal direction of the vessel (3) either continuously or in pulses.

3. The system according to claim 1 or 2, which further comprises a magnetizing device for magnetizing a bodily fluid (1), particularly blood.

4. The system according to claim 3, wherein the magnetizing device is designed to inject magnetized or magnetizable particles (1.1) into the bodily fluid (1).

5. The system according to claim 3, wherein the magnetizing device is designed to treat the bodily fluid (1) in such a way that a dipole moment is formed in constituent elements (1.1) of the bodily fluid (1).

6. The system according to one of claims 3 to 5, wherein the magnetizing device is designed to extravascularly, intravascularly or intracorporeally magnetize the bodily fluid (1), in particular by means of oral administration or by means of intravenous injection of magnetized or magnetizable particles (1.1).

7. The system according to one of claims 3 to 5, wherein the magnetizing device is designed to extracorporeally magnetize the bodily fluid (1), in particular by injection or diffusion of magnetized or magnetizable particles (1.1).

8. The system according to one of claims 1 to 7, wherein the device (2) for applying a magnetic field comprises a magnetic coil assembly at least sectionally arranged in the longitudinal direction of the vessel (3) which is segmented into a plurality of successive and independently actuable regions (2.1, 2.2, 2.3).

9. The system according to claim 8, wherein the control device is designed to successively actuate the individual regions (2.1. 2.2, 2.3) of the magnetic coil assembly in the direction of flow of the bodily fluid (1) such that one respective individual region (2.1, 2.2, 2.3) alone applies a magnetic field to the magnetized bodily fluid (1) in each case.

10. The system according to claim 8 or 9,
wherein the individual regions (2.1, 2.2, 2.3) of the magnetic coil assembly are spatially separated from one another; and wherein the first region (2.1) of the magnetic coil assembly as seen in the direction of flow of the bodily fluid (1) can be actuated by the control device in order to exert a magnetic torque on magnetized or magnetizable particles (1.1) disposed upstream of the first region, and wherein a further region (2.2, 2.3) of the magnetic coil assembly arranged downstream with respect to the first region (2.1) of the magnetic coil assembly is actuated by the control device when the magnetized or magnetizable particles (1.1) are moved along the vessel (3) in the direction of the first region (2.1) of the magnetic coil assembly by the magnetic field of said first region (2.1) of the magnetic coil assembly.

11. The system according to one of claims 1 to 10, wherein the device (2) for applying a magnetic field comprises a plurality of magnetic coil assemblies in successive vessel sections, and wherein a magnetic field applied by the device (2) is generated along the successive vessel sections such that a magnetic force having a component pointing in the longitudinal direction of the vessel (3) is exerted on magnetized or magnetizable particles (1.1) in the bodily fluid (1).

12. The system according to one of claims 1 to 11, wherein at least the device (2) for applying a magnetic field can be implanted into the body of a patient.

## Revendications

1. Système d'assistance circulatoire ventriculaire, le système comprenant ce qui suit :
- un moyen (2) d'application d'un champ magnétique à un liquide corporel magnétisé (1), en particulier du sang, dans une zone d'un vaisseau, en particulier d'un vaisseau sanguin ; et
- un moyen de commande qui est réalisé pour piloter le moyen (2) d'application d'un champ magnétique de telle sorte qu'une force induite par voie magnétique agit en direction longitudinale du vaisseau sur le liquide corporel magnétisé (1) dans la zone du vaisseau (3).

2. Système selon la revendication 1,
dans lequel
le moyen de commande est en outre réalisé pour varier dans le temps l'intensité et/ou la direction du champ magnétique appliqué sur le liquide corporel magnétisé (1), de telle sorte que le liquide corporel (1) se déplace de préférence sélectivement en continu ou par impulsions en direction longitudinale du vaisseau (3).

3. Système selon la revendication 1 ou 2, comprenant en outre un moyen de magnétisation pour magnétiser un liquide corporel (1), en particulier du sang.

4. Système selon la revendication 3,
dans lequel
le moyen de magnétisation est réalisé pour injecter des particules magnétisées ou magnétisables (1.1) dans le liquide corporel (1).

5. Système selon la revendication 3,
dans lequel
le moyen de magnétisation est réalisé pour traiter le liquide corporel (1) de telle sorte qu'un moment dipolaire est réalisé dans des constituants (1.1) du liquide corporel.

6. Système selon l'une des revendications 3 à 5,
dans lequel
le moyen de magnétisation est réalisé pour magnétiser le liquide corporel (1) par voie extravasculaire, intra-vasculaire ou intracorporelle, en particulier par administration orale ou par injection intraveineuse de particules magnétisées ou magnétisables (1.1).

7. Système selon l'une des revendications 3 à 5,
dans lequel
le moyen de magnétisation est réalisé pour magnétiser le liquide corporel (1) par voie extracorporelle, en particulier par injection ou par diffusion de particules magnétisées ou magnétisables (1.1).

8. Système selon l'une des revendications 1 à 7,
dans lequel
le moyen (2) d'application d'un champ magnétique comprend un ensemble à bobine magnétique agencé au moins localement en direction longitudinale du vaisseau (3), qui est segmenté en plusieurs zones (2.1, 2.2, 2.3) successives pilotables indépendamment les unes des autres.

9. Système selon la revendication 8,
dans lequel
le moyen de commande est réalisé pour piloter successivement les zones individuelles (2.1, 2.2, 2.3) de l'ensemble à bobine magnétique en direction de circulation du liquide corporel (1), de telle sorte qu'un champ magnétique est appliqué au liquide corporel magnétisé (1) par une seule zone individuelle respective (2.1, 2.2, 2.3).

10. Système selon la revendication 8 ou 9,
dans lequel
les zones individuelles (2.1, 2.2, 2.3) de l'ensemble à bobine magnétique sont séparées dans l'espace les unes des autres, et
vue en direction de circulation du liquide corporel (1), la première zone (2.1) de l'ensemble à bobine magnétique est pilotable par le moyen de commande pour exercer un moment magnétique sur les particules magnétisées ou magnétisables (1.1) disposées en amont de la première zone, et
une autre zone (2.2, 2.3) de l'ensemble à bobine magnétique, disposée en aval de la première zone (2.1) de l'ensemble à bobine magnétique, est pilotée par le moyen de commande lorsque les particules magnétisées ou magnétisables (1.1) ont été déplacées par le champ magnétique de la première zone (2.1) de l'ensemble à bobine magnétique le long du vaisseau (3) en direction de la première zone (2.1) de l'ensemble à bobine magnétique.

11. Système selon l'une des revendications 1 à 10,
dans lequel
le moyen (2) d'application d'un champ magnétique présente une multitude d'ensembles à bobine magnétique dans des portions de vaisseau successives, et
un champ magnétique appliqué par le moyen (2) est généré le long des portions de vaisseau successives de telle sorte qu'une force magnétique est exercée sur des particules magnétisées ou magnétisables (1.1) dans le liquide corporel, laquelle présente une composante dirigée en direction longitudinale du vaisseau (3).

12. Système selon l'une des revendications 1 à 11,
dans lequel
le moyen (2) d'application d'un champ magnétique est implantable dans le corps d'un patient.
